# EUROPEAN PATENT SPECIFICATION

(11) **EP 4 052 744 B1**
(45) Date of publication and mention of the grant of the patent: **11.02.2026**
(21) Application number: 20881002.8
(22) Date of filing: 29.10.2020
(51) Int. Cl.: A61M 37/00, A61M 5/142, A61M 5/30

(54) **MULTI-SHOT INJECTION PATCH**
INJEKTIONSPFLASTER FÜR MEHRERE IMPFUNGEN
TIMBRE MULTI-INJECTION

(30) Priority: 30.10.2019 US 201962928266 P
(43) Date of publication of application: 07.09.2022
(73) Proprietor: Daicel Corporation, Osaka 530-0011 (JP)
(72) Inventor: Forman, David Michael, Fountain Hills, Arizona 85268 (US)
(74) Representative: Grünecker Patent- und Rechtsanwälte PartG mbB
(86) International application number: PCT/JP2020/040706
(87) International publication number: WO 2021/085565

(56) References cited:
- JP-A- 2006 524 120
- JP-A- 2010 504 797
- US-A- 3 308 818
- US-A- 5 860 957
- US-A- 5 860 957
- US-A1- 2003 114 789
- US-A1- 2006 216 337
- US-A1- 2006 216 337
- US-B1- 6 258 063

## Description

### Technical Field

The present disclosure relates to apparatuses and methods for needle-free injection of medication. More specifically, the apparatuses and methods relate to a wearable device that provides multiple needle-free injections of the medication through the skin of a patient.

### Background Art

Needle-free injection systems are known. The motive force employed by these systems include pyrotechnics, springs, or electromagnetic actuators (see, for example, Patent document 1). However, the devices of these systems are of a single-shot design and have a significant length that makes them ill-suited as a wearable device. Wearable multi-shot injection patches are known from the Patent Documents 2 and 3 cited below.

### Citation List

### Patent Document

Patent Document 1: JP 2016-49246 A / Patent Document 2: US 2006/216337 A1 / Patent Document 3: US 5 860 957 A

### Summary of Invention

### Technical Problem

In certain medical applications, it can be advantageous to utilize a needle-free injection device. Certain embodiments of the invention provide multiple, needle-free, injection devices mounted on a single wearable patch. For example, a doctor may require that a patient receives an injection of medicine every 3 hours for 3 days. Currently, if the patient is unable to inject themselves, or if the type of injection is not suitable for a patient to self-administer, then a health professional will need to make frequent visits to the patient, or else the patient may need to make frequent visits to a health clinic, or the patient may need to be hospitalized while receiving treatment. Embodiments of this invention permit the required injections to be done automatically, while the patient is wearing the invented device and possibly going about their normal daily activities.

### Solution to Problem

In certain embodiments, each injection device is actuated by an electric current igniting a pyrotechnic charge within the injection device. In certain embodiments, electronics are employed to control the triggering or firing of each injection device. In certain embodiments, the electronics are capable of triggering or firing each injection device independently. In certain embodiments, the patch is compact and thin enough to be worn by the patient so that the injections can be administered remotely or in a predetermined timed sequence.

In certain embodiments, the patch is around 5 mm to 10 mm thick. In certain embodiments, the patch is suitable as a wearable device. In certain embodiments where each injection device is about 5 mm to 10 mm in diameter, several injection devices may be placed on the same wearable patch.

The invention comprises a wearable multi-shot injection patch according to claim 1, and a method for manufacturing a wearable multi-shot injection patch according to claim 16.

In some embodiments, a wearable multiple shot injection patch can include a base having a bottom surface and a top surface. The bottom surface is configured for placement against skin of a patient. The patch further includes a first receptacle supported by the base that contains a first medication and a second receptacle supported by the base that contains a second medication. A first pyrotechnic charge is disposed in the base and between the top surface and the first receptacle. A second pyrotechnic charge is disposed in the base and between the top surface and the second receptacle. One or more electronic components are configured to trigger the first pyrotechnic charge and the second pyrotechnic charge. A first nozzle is supported by the base and disposed on an opposite side of the first receptacle from the first pyrotechnic charge.

A second nozzle is supported by the base and disposed on an opposite side of the second receptacle from the second pyrotechnic charge.

In some embodiments, a wearable multiple shot injection patch can include a base having a bottom surface and a top surface. The bottom surface is configured for placement against skin of a patient. Two or more receptacles are supported by the base and contain a solution including one or more drugs and other ingredients. The two or more receptacles release the solution simultaneously or sequentially by using pyrotechnic charge initiated by electric pulse to penetrate the skin of the patient.

In some embodiments, a wearable injection patch can include a base having a bottom surface and a top surface. The bottom surface is configured for placement against skin of a patient. A receptacle is formed by an outer wall and contains a medication. The outer wall is supported by the base. A pyrotechnic charge is disposed in the base and between the top surface and the outer wall of the receptacle.

In some embodiments, a method for manufacturing a wearable multiple shot injection patch can include providing a first subassembly having a bottom surface and a top surface and comprising a plurality of receptacles disposed between the bottom surface and the top surface. The bottom surface is configured for placement against skin of a patient. Each receptacle of the plurality of receptacles contains a medication. The method continues by providing a second subassembly comprising a plurality of chambers. Each chamber of the plurality of chambers includes a pyrotechnic charge. The method continues by joining the second subassembly to the top surface of the first subassembly so that the plurality of chambers are on an opposite side of the plurality of receptacles from the bottom surface.

In some embodiments, a method for injecting a medication through skin of a patient using a wearable patch having a bottom surface and a top surface can include adhering the bottom surface of the wearable patch to the skin of the patient, the patch comprising a plurality of receptacles and a plurality of chambers aligned with the plurality of receptacles, the plurality of receptacles being disposed between the bottom surface and the top surface with each receptacle of the plurality of receptacles containing the medication, each chamber of the plurality of chambers being disposed between the plurality of receptacles and the top surface and comprising a pyrotechnic charge; and triggering the pyrotechnic charge in at least one chamber of the plurality of chambers to compress at least one receptacle of the plurality of receptacles so as to eject the medication from the at least receptacle and through the skin of the patient.

In some embodiments, a wearable multiple shot injection patch can include a base having a bottom surface and a top surface, the bottom surface being configured for placement against skin of a patient; a first receptacle supported by the base and containing a first medication; a second receptacle supported by the base and containing a second medication; a first pyrotechnic charge disposed in the base and between the top surface and the first receptacle; and a second pyrotechnic charge disposed in the base and between the top surface and the second receptacle.

### Advantageous Effects of Invention

According to the technique according to the present disclosure, it is possible to provide a wearable injection patch for performing needle-free injections of medication through the skin of a patient.

### Brief Description of Drawings

Fig. 1 is a perspective view of an embodiment of a patch that includes a plurality of injection devices distributed across the patch according to a preferred embodiment of the present invention.
Fig. 2 is a top view of the patch from Fig. 1 illustrating one or more electronic components for triggering a plurality of pyrotechnic charges for the plurality of injection devices.
Fig. 3 is a perspective exploded view of the patch from Fig. 1 illustrating two or more receptacles storing solution aligned below a clamp sheet and above a sheet, a mask layer, and the plurality of pyrotechnic charges.
Fig. 4 is a cross-sectional view of the patch of Fig. 2, as viewed along the cut-plane 4-4 of Fig. 2, illustrating nozzles of three of the plurality of injection devices covered by a removable release liner prior to the patch being attached to skin of a patient.
Fig. 5 is a cross-sectional view similar to Fig. 4 except the release liner has been removed and one of the plurality of pyrotechnic charges has been triggered expelling the solution from its nozzle into the skin of the patient.
Fig. 6 is a perspective exploded view of a first subassembly of the patch that includes the one or more electronic components, the plurality of pyrotechnic charges, the mask layer, and the sheet.
Fig. 7A is a cross-sectional view of the first subassembly from Fig. 6, as viewed along the cut-plane 7A-7A of Fig. 6, illustrating a circuit board that supports one of the plurality of pyrotechnic charges being assembled to a lower surface of the mask layer. The sheet is illustrated being assembled to a top surface of the mask layer so that one of a plurality of diaphragms of the sheet aligns with one of the plurality of chambers of the mask layer.
Fig. 7B is a cross-sectional view similar to Fig. 7A except the circuit board has been assembled to the lower surface of the mask layer and the sheet has been assembled to the top surface of the mask layer.
Fig. 8 is a perspective exploded view of a second subassembly of the patch that includes the clamp sheet and the two or more receptacles. The two or more receptacles are aligned with recesses in the clamp sheet. The removable release liner is illustrated disposed above the clamp sheet.
Fig. 9A is a cross-sectional view of the second subassembly from Fig. 8, as viewed along the cut-plane 9A-9A of Fig. 8, illustrating one of the two or more receptacles being assembled into one of the recesses in the clamp sheet. The removable release liner is illustrated above the clamp sheet.
Fig. 9B is a cross-sectional view similar to Fig. 9A except the receptacle has been inserted into the recess in the clamp sheet and the release liner has been attached to the clamp sheet.
Fig. 10 illustrates another embodiment of the receptacle in Fig. 4 that has a truncated triangular shape.
Fig. 11 illustrates another embodiment of the receptacle in Fig. 4 that has a truncated pentagon shape.

### Description of Embodiments

Embodiments according to the present disclosure will be described below with reference to the drawings. Note that each of the configurations, combinations thereof, and the like in each embodiment is an example, and additions, omissions, substitutions, and other changes of the configuration may be made as appropriate without departing from the present invention. The present disclosure is not limited by the embodiments and is limited only by the claims.

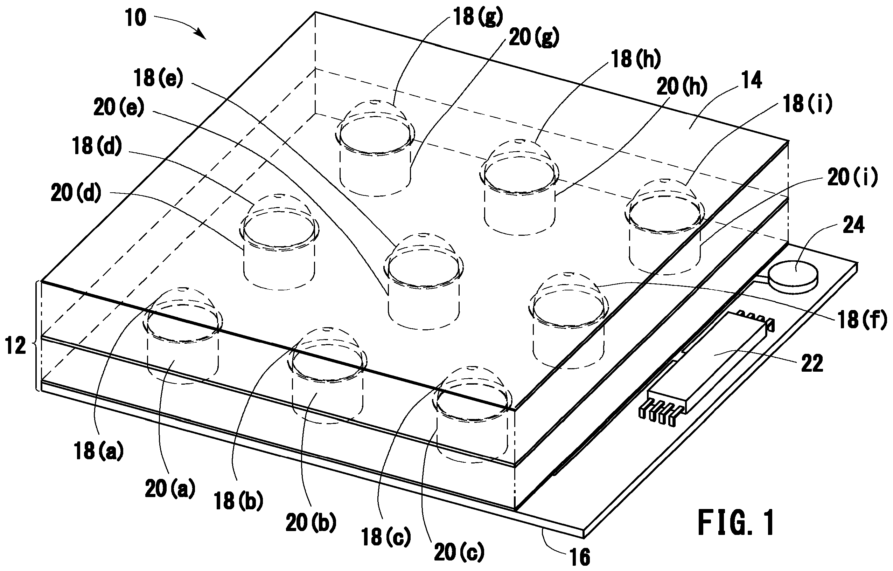

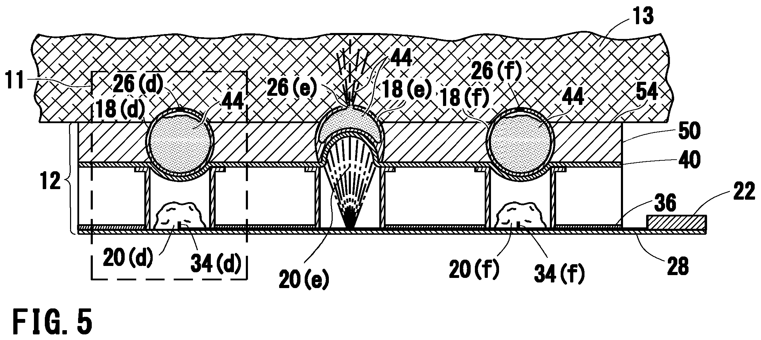

Fig. 1 is a perspective view of an embodiment of a patch 10 that includes a plurality of injection devices 11 (see Fig. 5) distributed across the patch 10 according to a preferred embodiment of the present invention. In certain embodiments, the patch 10 includes a base 12 having a bottom surface 14 and a top surface 16. The bottom surface 14 is configured for placement against skin 13 of a patient as best seen in Fig. 5.

In certain embodiments, the patch 10 includes two or more receptacles 18(a)-(i). In certain embodiments, the two or more receptacles 18(a)-(i) are supported by the base 12. In certain embodiments, the two or more receptacles 18(a)-(i) contain a medication. In certain embodiments, the medication is a solution 44. In certain embodiments, the solution 44 includes one or more drugs and other ingredients. In certain embodiments, the medication or solution 44 in more than one of the receptacles 18(a)-(i) is the same. In certain embodiments, the medication or solution 44 in the two or more receptacles 18(a)-(i) is different. In certain embodiments, the medication or solution 44 in the two or more receptacles 18(a)-(i) is different but complementary to each other.

In certain embodiments, each of the two or more receptacles 18(a)-(i) include a flexible spherical-like vessel for receiving the solution 44. In certain embodiments, the solution 44 is received by injecting the solution 44 into the two or more receptacles 18(a)-(i). In certain embodiments, the solution 44 is injected into the two or more receptacles 18(a)-(i) prior to assembly of the patch 10. In certain embodiments, the solution 44 is injected into the two or more receptacles 18(a)-(i) during assembly of the patch 10. In certain embodiments, the solution 44 is injected into the two or more receptacles 18(a)-(i) after assembly of the patch 10.

In certain embodiments, the patch 10 includes a plurality of pyrotechnic charges 20(a)-(i). In certain embodiments, the plurality of pyrotechnic charges 20(a)-(i) are supported by the base 12. The plurality of pyrotechnic charges 20(a)-(i) can include any suitable ignition material. In certain embodiments, each of the plurality of pyrotechnic charges 20(a)-(i) includes a mixture of zirconium and potassium perchlorate. In certain embodiments, the mixture further includes a binder. In certain embodiments, the plurality of pyrotechnic charges 20(a)-(i) are deposited as a wet slurry and then dried.

The plurality of pyrotechnic charges 20(a)-(i) are configured to release the solution 44 from the two or more receptacles 18(a)-(i) in a controlled manner. In certain embodiments, the vessels of the two or more receptacles 18(a)-(i) are desirably flexible enough to permit the burning and expansion of the pyrotechnic charge 20 to deform the vessel sufficiently to eject the solution 44 or medication through one or more nozzles 26(a)-(i) associated with each of the two or more receptacles 18(a)-(i). When released, the solution 44 desirably penetrates the skin 13 of the patient. In certain embodiments, the plurality of pyrotechnic charges 20(a)-(i) release the solution 44 from a plurality of the two or more receptacles 18(a)-(i) simultaneously. In certain embodiments, the plurality of pyrotechnic charges 20(a)-(i) release the solution 44 from a plurality of the two or more receptacles 18(a)-(i) sequentially.

In certain embodiments, the solution 44 released by the plurality of pyrotechnic charges 20(a)-(i) penetrates the skin 13 to a desired depth into the skin 13. In certain embodiments, the desired depth is predefined. In certain embodiments, the desired depth is determined at least in part based on the composition of the released medication or solution 44. For example, the desired depth can be set to a different depth for each injection device 11 on the same patch 10.

In certain embodiments, the patch 10 includes one or more electronic components 22 for triggering the plurality of pyrotechnic charges 20(a)-(i). In certain embodiments, the one or more electronic components 22 are supported by the base 12.

In certain embodiments, the one or more electronic components 22 are configured to generate an electric current to trigger the plurality of pyrotechnic charges 20(a)-(i). In certain embodiments, the one or more electronic components 22 are configured to selectively trigger the plurality of pyrotechnic charges 20(a)-(i). In certain embodiments, the one or more electronic components 22 are configured to independently trigger each of the plurality of pyrotechnic charges 20(a)-(i). In certain embodiments, the one or more electronic components 22 are configured to simultaneously trigger more than one of the plurality of pyrotechnic charges 20(a)-(i).

In certain embodiments, the one or more electronic components 22 are configured to be controlled remotely. In certain embodiments, the one or more electronic components 22 are configured to be controlled in a predetermined timed sequence.

In certain embodiments, the one or more electronic components 22 include a controller. In certain embodiments, the controller is a microcontroller. In certain embodiments, the controller is programmable. In certain embodiments, the one or more electronic components 22 are programmed with a required number of injections, an injection interval, and the suitable receptacle 18(a)-(i) that contains the desired medication or solution 44. In certain embodiments, once applied to the skin 13 of the patient, the patch 10 automatically administers the medication without intervention from the health professional.

In certain embodiments, the controller is configured to store instructions to trigger at least one of the plurality of pyrotechnic charges 20(a)-(i). In certain embodiments, the instructions specify an interval between triggering charges of the plurality of pyrotechnic charges 20 (a)-(i). In certain embodiments, the one or more electronic components 22 include a timer. In such an embodiment, the controller can be configured to trigger at least one of the plurality of pyrotechnic charges 20(a)-(i) at least in part based on the timer.

In certain embodiments, the one or more electronic components 22 includes a receiver configured to receive a wireless signal. In such an embodiment, the one or more electronic components 22 can be further configured to trigger at least one of the plurality of pyrotechnic charges 20(a)-(i) based at least in part on the wireless signal. In certain embodiments, the receiver is configured to receive the wireless signal from the patient. In certain embodiments, the receiver is configured to receive the wireless signal from the health professional.

In certain embodiments, the patch 10 includes a user interface configured for the patient to initiate the one or more electronic components 22 which triggers at least one of the plurality of pyrotechnic charges 20(a)-(i).

In certain embodiments, the patch 10 includes an energy source for the one or more electronic components 22. In certain embodiments, the energy source is supported by the base 12. In certain embodiments, the energy source can be a battery 24. In certain embodiments, the battery 24 powers the one or more electronic components 22 to fire a selected injection device 11. In certain embodiments, the one or more electronic components 22 provide a first electric pulse to a selected one or more of the plurality of pyrotechnic charges 20(a)-(i) during a first time frame and then provide a second electric pulse to another selected one or more of the plurality of pyrotechnic charges 20(a)-(i) during a second time frame.

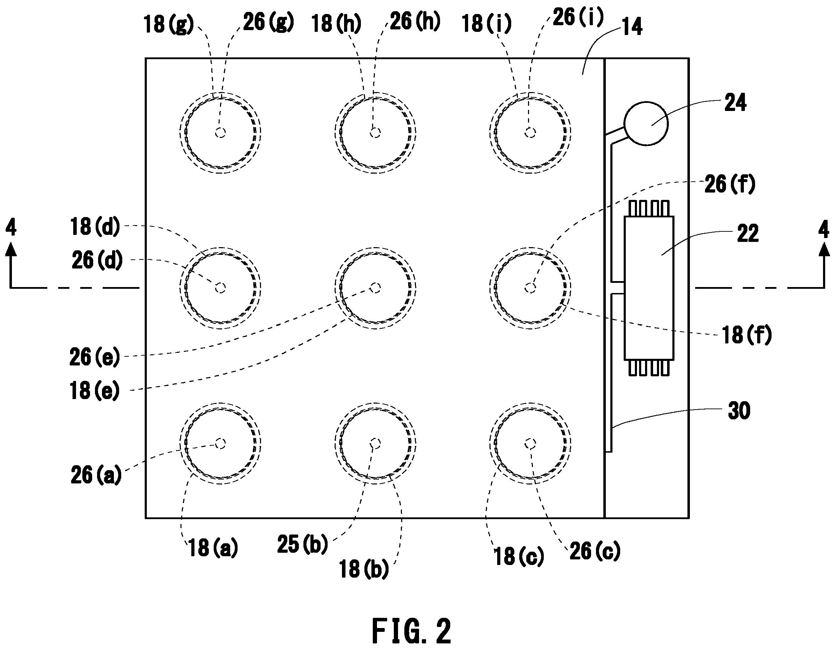

Fig. 2 is a top view of the patch 10 from Fig. 1 illustrating the one or more electronic component 22 for triggering the plurality of pyrotechnic charges 20(a)-(i) for the plurality of injection devices 11.

In the illustrated embodiment, the patch 10 includes nine injection devices 11 arranged in a grid pattern. Of course, the disclosure is not so limited. The patch 10 can include as few as two injection devices 11. In other embodiments, the patch 10 includes more than nine injection devices 11.

In the illustrated embodiment, the two or more receptacles 18(a)-(i) have the same size. Of course, the disclosure is not so limited. In certain embodiments, at least one receptacle 18(a)-(i) has a different size than another receptacle 18(a)-(i). In certain embodiments, the size is a measure of an outer diameter of the receptacle 18(a)-(i). In certain embodiments, the size is a measure of an inner diameter of the receptacle 18(a)-(i). In certain embodiments, the size is a measure of a volume of the receptacle 18(a)-(i).

In certain embodiments, the volume of the receptacle 18(a)-(i) is selected at least partially on the medication or solution 44 contained within the receptacle 18(a)-(i). For example, a first medication may require a larger dose to be effective than a second medication. To accommodate variations in the size of doses, a single patch 10 can include receptacles 18(a)-(i) that have different volumes. In certain embodiments where the patch 10 includes receptacles 18(a)-(i) of a similar size and there is a need for variations in the size of the doses, the one or more electronic components 22 can trigger more than one of the plurality of pyrotechnic charges 20(a)-(i) to increase the size of the single dose.

The injection devices 11 can be arranged in symmetric or asymmetric patterns across the base 12 of the patch 10. For example, in certain embodiments, the injection devices 11 are arranged in a square pattern, a circular pattern, or any other spatial arrangement across the base 12 of the patch 10.

In certain embodiments, clusters of injection devices 11 are arranged across the base 12 of the patch 10. In certain embodiments, the injection devices 11 within a single cluster are grouped in a linear fashion. In other embodiments, the injection devices 11 within a single cluster are closely grouped at a single location on the patch 10 such as in a circle.

In the illustrated embodiment, the one or more electronic components 22 including the battery 24 are disposed on an outer perimeter of the patch 10. In other embodiments, the one or more electronic components 22 are embedded in the base 12 between the injection devices 11. In other embodiments, the one or more electronic components 22 are disposed on the top surface 16 of the patch 10. In other embodiments, the one or more electronic components 22 are disposed on a side of the base 12.

As illustrated in Fig. 2 in certain embodiments, each of the two or more receptacles 18(a)-(i) includes one of the nozzles 26(a)-(i). In certain embodiments, the nozzles 26(a)-(i) are supported by the base 12. In certain embodiments, the nozzles 26(a)-(i) are disposed on an opposite side of the two or more receptacles 18(a)-(i) from the plurality of pyrotechnic charges 20(a)-(i). In the illustrated embodiment, the nozzles 26(a)-(i) are disposed in a grid pattern across the patch 10.

As illustrated in Fig. 2, each nozzle 26(a)-(i) is aligned with one of the receptacles 18(a)-(i). In certain embodiments, each nozzle 26(a)-(i) is in fluid communication one of the two or more receptacles 18(a)-(i). In certain embodiments, two or more nozzle 26(a)-(i) are in fluid communication with one of the two or more receptacles 18(a)-(i). In certain other embodiments, each nozzle 26(a)-(i) is in fluid communication with two or more of the receptacles 18(a)-(i). For example, a pyrotechnic charge 20 for a first injection device 11 ejects medication or solution 44 from one of the receptacles 18 through nozzle 26(a) and then later a second injection device 11 ejects medication or solution 44 from another one of the receptacles 18 through the nozzle 26(a). For example, a pyrotechnic charge 20 for a first injection device 11 ejects medication or solution 44 from one of the receptacles 18 through the nozzle 26(a) at the same time a second and likely adjacent injection device 11 ejects medication or solution 44 from another one of the receptacles 18 through the nozzle 26(a).

The nozzles 26(a)-(i) can extend at least partially beyond a downstream end of the two or more receptacles 18(a)-(i). The nozzles 26(a)-(i) can be configured to output medication or solution 44 from the two or more receptacles 18(a)-(i). In some embodiments, the nozzles 26(a)-(i) can output medication or solution 44 in a pressurized manner. For example, the nozzles 26(a)-(i) can direct pressurized medication or solution 44 towards the skin 13 of the patient.

In certain embodiments, the sizes of the nozzles 26(a)-(i) are selected at least in part based on the desired depth for the medication to penetrate the skin 13. For example, the sizes of the nozzles 26(a)-(i) can be selected to achieve a desired velocity of a jet of liquid medication or solution 44 exiting the nozzle 26(a)-(i) that will achieve the desired depth penetrating the skin 13. In certain embodiments, a diameter of the nozzles 26(a)-(i) is less than a diameter of the two or more receptacles 18(a)-(i) at least when the plurality of pyrotechnic charges 20(a)-(i) has not been triggered.

In some embodiments, the nozzles 26(a)-(i) can output the medication or solution 44 in a predetermined direction. For example, the nozzles 26(a)-(i) can output the medication or solution 44 along a longitudinal axis of the nozzles 26(a)-(i). In some embodiments, the nozzles 26(a)-(i) can direct the medication or solution 44 towards a predetermined location on the skin 13 of the patient. In some embodiments, more than one of the nozzles 26(a)-(i) is aligned so as to direct the medications or solutions 44 toward the same location on the skin 13 of the patient.

For example in certain embodiments, a first nozzle 26 is positioned so that when the one or more electronic components 22 trigger a first pyrotechnic charge 20 at least a portion of a first medication 44 exits the first nozzle 26 and penetrates the skin 13 of the patient. A second nozzle 26 is positioned so that when the one or more electronic components 22 trigger the second pyrotechnic charge 20 at least a portion of a second medication 44 exits the second nozzle 26 and penetrates the skin 13 of the patient. In certain embodiments, the first nozzle 26 and the second nozzle 26 are disposed relative to each so that at least a portion of the first medication 44 penetrates the skin 13 of the patient at a location different from a location where at least a portion of the second medication 44 penetrates the skin 13 of the patient. In certain other embodiments, the first nozzle 26 and the second nozzle 26 are disposed relative to each so that at least a portion of the first medication 44 penetrates the skin 13 of the patient at a same location as at least a portion of the second medication 44 penetrates the skin 13 of the patient.

In certain embodiments, the nozzles 26(a)-(i) are manufactured as an integral component with the two or more receptacles 18(a)-(i). For example, the nozzles 26(a)-(i) can be formed by openings through walls of the two or more receptacles 18(a)-(i). In alternate embodiments, the nozzles 26(a)-(i) are manufactured as a separate component from the two or more receptacles 18(a)-(i) and subsequently positioned relative to the two or more receptacles 18(a)-(i). In certain embodiments, the nozzles 26(a)-(i) are positioned relative to the two or more receptacles 18(a)-(i) by bonding, ultrasonically welding, or clamping the nozzles 26(a)-(i) to the two or more receptacles 18(a)-(i). In certain embodiments, a separately manufactured nozzle 26 can be positioned relative to the opening through the wall of the receptacle 18. In such an embodiment, the inner diameter of the nozzle 26 can be smaller than a diameter of the opening in the wall of the receptacle 18 so as to provide a more narrow flow path which accelerates the jet of medication or solution 44 leaving the receptacle 18.

In certain embodiments, the nozzles 26(a)-(i) have constant internal diameters. In certain embodiments, the nozzles 26(a)-(i) have converging internal diameters in a direction of flow. In certain embodiments, the nozzles 26(a)-(i) have diverging internal diameters in the direction of flow. In certain embodiments, the nozzles 26(a)-(i) have converging and then diverging internal diameters in the direction of flow.

In certain embodiments, the nozzles 26(a)-(i) can have one or more vanes formed in the internal flow path. In certain embodiments, the one or more vanes are sized and shaped to straighten the solution 44 passing through the nozzle 26. The vanes can reduce turbulence in the solution 44 as the solution 44 is ejected from the nozzle 26. In certain embodiments, the nozzle 26 is formed as a screen. In certain embodiments, the screen has a plurality of pores. In certain embodiments, the plurality of pores are micro pores.

In the illustrated embodiment, each of the two or more receptacles 18(a)-(i) includes a single nozzle 26. Of course, the disclosure is not so limited. In certain embodiments, one receptacle 18 can include a plurality of nozzles 26. In embodiments where the receptacle 18 is associated with a plurality of nozzles 26, each nozzle 26 may have the same or different size than another of the nozzles 26 associated with the same receptacle 18.

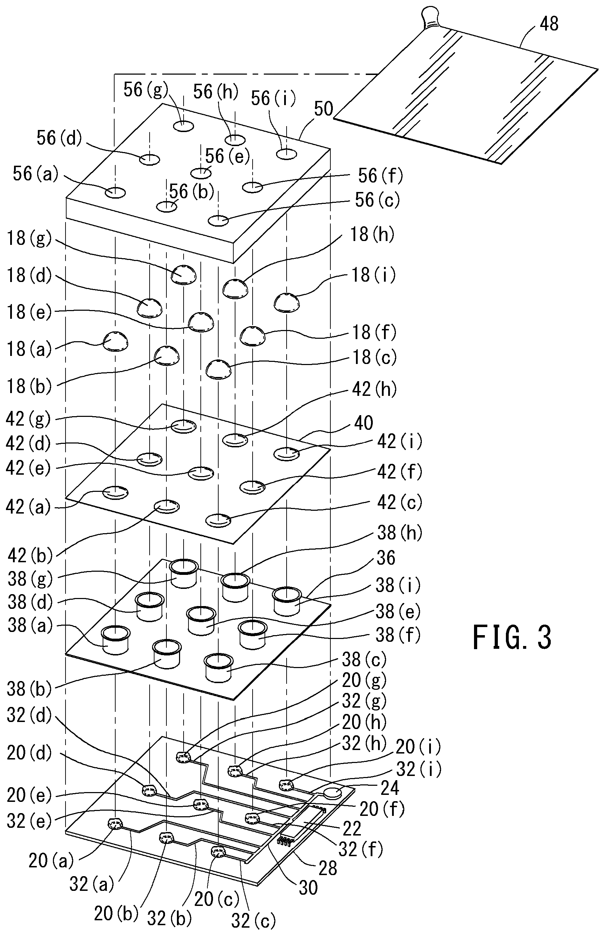

Fig. 3 is a perspective exploded view of the patch 10 from Fig. 1 illustrating the two or more receptacles 18(a)-(i) before their insertion into a clamp sheet 50. Fig. 3 further illustrates the stacked arrangement of the clamp sheet 50, a sheet 40, and a mask layer 36 above the plurality of pyrotechnic charges 20(a)-(i). A removable release liner 48 is illustrated disposed above the patch 10.

In certain embodiments, the patch 10 includes a printed circuit board (PCB) or circuit board 28 supporting the plurality of pyrotechnic charges 20(a)-(i). In certain embodiments, the circuit board 28 is flexible so that the bottom surface 14 of the base 12 can conform to a surface of a limb or torso of the patient. In certain embodiments, the circuit board 28 need not be flexible. For example, for locations on the patient that are less curved, the circuit board 28 need only be curved or flexible enough to contact the skin 13. For example, a small patch 10 may be less flexible or curved since the contact surface on the skin 13 is similarly small. In certain embodiments, the circuit board 28 further supports the one or more electronic components 22.

In certain embodiments, the circuit board 28 includes at least one trace 32(a)-(i) extending between the one or more electronic components 22 and each of the plurality of pyrotechnic charges 20(a)-(i). In certain embodiments, the at least one trace 32(a)-(i) is configured to allow the one or more electronic components 22 to individually trigger each of the plurality of pyrotechnic charges 20(a)-(i). In certain embodiments, the one or more electronic components 22 and the plurality of pyrotechnic charges 20(a)-(i) are collocated so that the one or more electronic components 22 directly electrically connect to the plurality of pyrotechnic charges 20(a)-(i) without employing the at least one trace 32(a)-(i).

In certain embodiments employing the at least one trace 32(a)-(i), the at least one trace 32(a)-(i) can follow any path across the circuit board 28 between the one or more electronic components 22 and the plurality of pyrotechnic charges 20(a)-(i). For example, in the illustrated embodiment, a portion 30 of the at least one trace 32(a)-(i) extends in a lateral direction from the one or more electronic components 22 before each trace 32 separately turns and extends in a generally longitudinal direction towards its respective pyrotechnic charge 20.

Each trace 32 (i.e., trace 32(a)) can include two distinct wires for completing at least a portion of an electrical circuit with the one or more electronic components 22. In certain embodiments, each pyrotechnic charge 20 is associated with two wires of each trace 32. The two traces 32 or wires form a portion of an electrical circuit between the one or more electronic components 22 and each charge of the plurality of pyrotechnic charges 20.

In certain embodiments, the patch 10 includes the clamp sheet 50. In certain embodiments, the clamp sheet 50 supports the two or more of receptacles 18(a)-(i). For example, in certain embodiments, the two or more receptacles 18(a)-(i) are inserted into individual recesses 56(a)-(i) in the clamp sheet 50. In certain embodiments, each recess 56(a)-(i) is formed as an inverted pocket in the clamp sheet 50. In certain embodiments, each pocket is sized and shaped to generally match an outer surface of the two or more receptacles 18(a)-(i).

In certain embodiments, openings at a top of the recess 56(a)-(i) (closest to the sheet 40) have a size that facilitates insertion of the two or more receptacles 18(a)-(i) into the recesses 56(a)-(i) in the clamp sheet 50 during assembly. In certain embodiments, the openings (at the tops of the recesses 56) have a diameter that is equal to or greater than a maximum diameter of the two or more receptacles 18(a)-(i). In certain other embodiments, the openings have a diameter that is slightly smaller than a maximum diameter of the two or more receptacles 18(a)-(i) but still allows insertion of the two or more receptacles 18(a)-(i) into the recesses 56(a)-(i) in the clamp sheet 50 during assembly.

In certain embodiments, a bottom surface (closest to the release liner 48) of each recess 56(a)-(i) includes an opening through the clamp sheet 50. In certain embodiments, the openings (on the bottom surfaces of the recesses 56) are sized smaller than a diameter of the two or more receptacles 18(a)-(i). In this way, the two or more receptacles 18(a)-(i) are inhibited from passing through the openings in the clamp sheet 50 when the plurality of pyrotechnic charges 20(a)-(i) deform the two or more receptacles 18(a)-(i) to eject the solution 44.

In certain embodiments, the patch 10 includes the sheet 40. In certain embodiments, the sheet 40 includes a polymer material. In certain other embodiments, the sheet 40 includes a non-polymer material. In certain embodiments, the sheet 40 is disposed on the opposite side of the two or more receptacles 18(a)-(i) from the clamp sheet 50. In certain embodiments, the sheet 40 is bonded by, for example, ultrasonic welding, bonding, clipping, and clamping to the clamp sheet 50. When assembled, the sheet 40 provides a barrier between the plurality of pyrotechnic charges 20(a)-(i) and the two or more receptacles 18(a)-(i).

In certain embodiments, the sheet 40 includes one or more diaphragms 42(a)-(i). In the illustrated embodiment, each diaphragm 42(a)-(i) is associated with one of the two or more receptacles 18(a)-(i). In certain embodiments, each diaphragm 42(a)-(i) is associated with more than one of the two or more receptacles 18(a)-(i). In certain embodiments, the sheet 40 includes a single diaphragm 42 and a perimeter portion surrounding the single diaphragm 42.

In certain embodiments, the diaphragms 42(a)-(i) are flexible. In certain embodiments, the diaphragms 42(a)-(i) are more flexible than the remainder of the sheet 40. In certain embodiments, the diaphragms 42(a)-(i) are as flexible as the remainder of the sheet 40. The flexibility of the diaphragms 42(a)-(i) may allow the diaphragms 42 to protrude into the recesses 56(a)-(i) in the clamp sheet 50 and compress the two or more receptacles 18(a)-(i) in response to a pressure spike generated by the plurality of pyrotechnic charges 20(a)-(i).

In certain embodiments, the sheet 40 is generally planar and has a consistent thickness across the sheet 40. In certain embodiments, the sheet 40 is generally planar except in the areas of the diaphragms 42(a)-(i). In certain embodiments, the thickness of the sheet 40 in the areas of the diaphragms 42(a)-(i) is less than the thickness of the remainder of the sheet 40. In certain embodiments, the areas of the diaphragms 42(a)-(i) have a dome shape. In certain embodiments, the areas of the diaphragms 42(a)-(i) have a shape that generally matches the shape of a portion of the two or more receptacles 18(a)-(i) that is adjacent to the diaphragm 42 when assembled.

In certain embodiments, the patch 10 includes the mask layer 36. In certain embodiments, the mask layer 36 forms a spacer between the circuit board 28 and the two or more receptacles 18(a)-(i). In certain embodiments, the mask layer 36 forms a barrier between adjacent pyrotechnic charges 20(a)-(i). In certain embodiments, the barrier prevents one injection device 11 from sympathetically actuating another injection device 11.

In certain embodiments, the mask layer 36 includes one or more chambers 38. In certain embodiments, each chamber 38 has a cylindrical shape. In certain other embodiments, each chamber 38 has a square shape or any other shape. In certain embodiments, each of the one or more chambers 38 is associated with one injection device 11. In the illustrated embodiment, each of the one or more chambers 38(a)-(i) is associated with one of the plurality of pyrotechnic charges 20(a)-(i).

In the illustrated embodiments, each of the one or more chambers 38(a)-(i) has an open top and an open bottom. In certain embodiments, one or both of the top and bottom of each of the one or more chambers 38(a)-(i) is closed. In the illustrated embodiment, the one or more chambers 38(a)-(i) extend in a direction away from the circuit board 28. In other embodiments, the mask layer 36 is flipped upside down relative to the embodiment illustrated in Fig. 3. In such an embodiment, the one or more chambers 38(a)-(i) extend in a direction towards the circuit board 28.

In certain embodiments, the mask layer 36 is bonded or otherwise attached to the circuit board 28 via ultrasonic welding, bonding, clipping, clamping or other attachment means. In certain embodiments, the mask layer 36 is compressed between the sheet 40 and the circuit board 28. In certain embodiments, the mask layer 36 is bonded or otherwise attached to the sheet 40 via ultrasonic welding, bonding, clipping, clamping or other attachment means. In certain embodiments, the mask layer 36 includes a polymer material. Of course, the disclosure is not so limited and the mask layer 36 can be made from non-polymer materials.

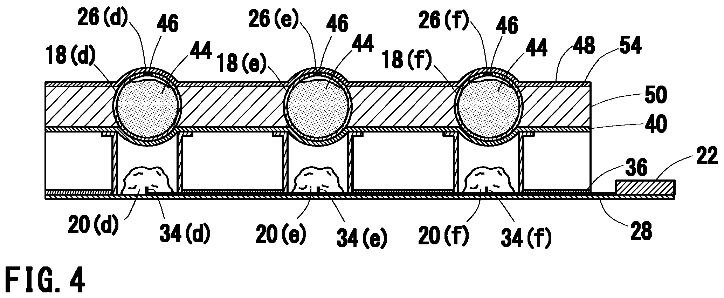

Fig. 4 is a cross-sectional view of the patch 10 of Fig. 2, as viewed along the cut-plane 4-4 of Fig. 2, illustrating nozzles 26(d), 26(e), 26(f) covered by the removable release liner 48 prior to the patch 10 being attached to the skin 13 of the patient. In certain embodiments, a seal 46 is disposed in the nozzles 26(a)-(i). The seal 46 inhibits the solution 44 from leaking out of the two or more receptacles 18(a)-(i) when the injection device 11 has not been triggered. Once triggered, the pressure in the two or more receptacles 18(a)-(i) caused by the compression of the receptacle 18 breaks the seal 46 allowing the solution 44 to pass through and exit the nozzle 26.

In certain embodiments, the patch 10 includes a plurality of resistance wires 34(a)-(i). Fig. 4 illustrates resistance wires 34(d), 34 (e), 34 (f). In certain embodiments, the resistance wires 34(a)-(i) are in contact with the plurality of pyrotechnic charges 20(a)-(i). In the illustrated embodiment, the resistance wire 34(d) is in contact with the pyrotechnic charge 20(d). In the illustrated embodiment, the resistance wire 34(e) is in contact with the pyrotechnic charge 20(e). In the illustrated embodiment, the resistance wire 34(f) is in contact with the pyrotechnic charge 20(f).

In certain embodiments, the resistance wires 34 (a)-(i) are further in electrical contact with the at least one trace 32 (see Fig. 3). In the illustrated embodiment, the resistance wire 34(d) is in contact with trace 32(d). In the illustrated embodiment, the resistance wire 34(e) is in contact with trace 32(e). In the illustrated embodiment, the resistance wire 34(f) is in contact with trace 32(f). As mentioned above, each trace 32 (i.e., trace 32(e)) can include two distinct wires for completing at least a portion of an electrical circuit with the one or more electronic components 22. For example, the one or more electronic components 22 can electrically connect to one end of each of the two wires of trace 32(d). Each end of the resistance wire 34(d) can then electrically connect to one of the other ends of each of the two wires of trace 32(d) completing the electrical circuit.

In certain embodiments, the resistance wire 34 has a diameter of 20 microns. In certain embodiments, the resistance wire 34 has a length of 0.5 mm. In certain embodiments, the resistance wire 34 has a resistance of 1 ohm. Of course, the disclosure is not so limited. The resistance wires 34(a)-(i) can have any length, diameter, and/or resistance value.

Fig. 5 is a cross-sectional view similar to Fig. 4 except the release liner 48 has been removed and one of the plurality of pyrotechnic charges 20(e) has been triggered expelling the solution 44 from its nozzle 26(e) into the skin 13 of the patient. In Fig. 5, the pressure from the burning pyrotechnic charge 20(e) moves or presses a portion of the receptacle 18(e) further into the recess 56 (a)-(i) of the clamp sheet 50 increasing the pressure in the receptacle 18(e). In this way, the pyrotechnic charge 20(e) provides pressure to push a jet of the solution 44 out through the nozzle 26(e) and into or through the skin 13. The burned pyrotechnic charge 20(e) itself does not pass through the nozzle 26(e) and into or through the skin 13.

In certain embodiments, the burned pyrotechnic charge 20(e) is contained within the patch 10. In certain other embodiments, the burned pyrotechnic charge 20(e) is allowed to escape through the nozzle 26(e) but its velocity is sufficiently attenuated so as to not enter the skin 13.

In certain embodiments, one or both of the sheet 40 and a wall of the receptacle 18(e) does not rupture when the solution 44 is ejected from the receptacle 18(e) and maintains a barrier between the burned pyrotechnic charge 20(e) and the skin 13 of the patient. In certain embodiments, both the sheet 40 and the wall of the receptacle 18(e) rupture when the solution 44 is ejected from the receptacle 18(e) but, due to for example, the size and shape of the rupture attenuates the velocity of escaping burned pyrotechnic charge 20(e) sufficiently so that the burned pyrotechnic charge 20(e) does not enter the skin 13. In such an embodiments, the source of the force causing the solution 44 to enter the skin 13 is still the momentum of the solution 44 itself and not incidental contact between any of the burned pyrotechnic charge 20(e) that may have leaked from the patch 10 and the skin 13.

In certain embodiments, a height of the chambers 38(a)-(i) is selected to provide a sufficient space between the plurality of pyrotechnic charges 20(a)-(i) and the sheet 40 that allows the pressure from the burning pyrotechnic charges 20(a)-(i) to adequately compress the two or more receptacles 18(a)-(i) ejecting the solution 44 without rupturing the two or more receptacles 18(a)-(i). In certain embodiments, the height of the chambers 38(a)-(i) is selected to provide a sufficient space between the plurality of pyrotechnic charges 20(a)-(i) and the sheet 40 that allows the pressure from the burning pyrotechnic charges 20 to adequately compress the two or more receptacles 18(a)-(i) ejecting the solution 44 without rupturing the sheet 40. In certain embodiments, the height of the chambers 38 is selected to provide a sufficient space between the plurality of pyrotechnic charges 20(a)-(i) and the sheet 40 that allows the pressure from the burning pyrotechnic charges 20 to adequately compress the two or more receptacles 18(a)-(i) ejecting the solution 44 without rupturing at least one of the sheet 40 and the two or more receptacles 18(a)-(i). In certain embodiments, the height of the chambers 38 is selected to provide a sufficient space between the plurality of pyrotechnic charges 20(a)-(i) and the sheet 40 that allows the pressure from the burning pyrotechnic charges 20 to adequately compress the two or more receptacles 18(a)-(i) ejecting the solution 44 while attenuating the pressure of any of the burning pyrotechnic charge 20 that may escape through ruptures in the sheet 40 and the receptacles 18(a)-(i).

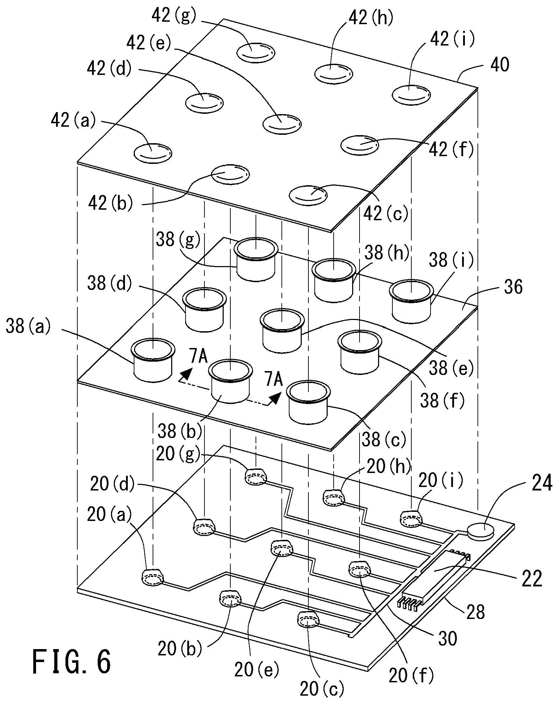

Fig. 6 is a perspective exploded view of a first subassembly of the patch 10 that includes the one or more electronic components 22, the plurality of pyrotechnic charges 20(a)-(i), the mask layer 36, and the sheet 40. The mask layer 36 includes the plurality of chambers 38(a)-(f) for the plurality of pyrotechnic charges 20(a)-(i).

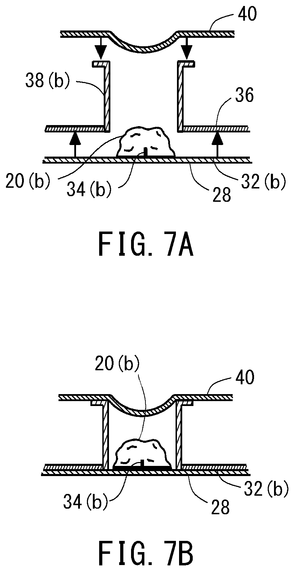

Fig. 7A is a cross-sectional view of the first subassembly from Fig. 6, as viewed along the cut-plane 7A-7A of Fig. 6, illustrating the circuit board 28 that supports one of the plurality of pyrotechnic charges 20(b) being assembled to a lower surface of the mask layer 36. The sheet 40 is illustrated being assembled to a top surface of the mask layer 36 so that the diaphragm 42(b) of the sheet 40 aligns with the chamber 38(b) of the mask layer 36.

Fig. 7B is a cross-sectional view similar to Fig. 7A except the circuit board 28 has been assembled to the lower surface of the mask layer 36 and the sheet 40 has been assembled to the top surface of the mask layer 36. In certain embodiments, one or all of the components of the first subassembly are joined together by, for example, ultrasonic welding, bonding, clipping, or clamping.

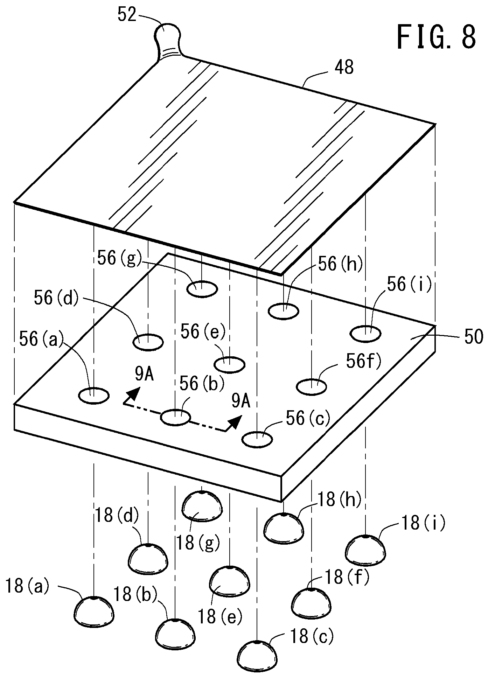

Fig. 8 is a perspective exploded view of a second subassembly of the patch 10 that includes the clamp sheet 50 and the two or more receptacles 18(a)-(i). In the illustrated embodiment, the two or more receptacles 18(a)-(i) are aligned with recesses 56(a)-(i) in the clamp sheet 50. The removable release liner 48 is illustrated disposed above the clamp sheet 50.

In certain embodiments, the patch 10 includes an adhesive layer 54. In certain embodiments, the adhesive layer 54 is configured to secure the patch 10 to the skin 13 of the patient. In certain embodiments, the adhesive layer 54 is configured to secure the patch 10 to the skin 13 of the patient for a duration of time.

In certain embodiments, the release liner 48 includes a tab 52 to ease removal of the release liner 48 from the clamp sheet 50. In certain embodiments, the patch 10 includes a strap coupled to the base 12 and configured to be secured around a limb or torso of the patient so as to maintain contact between the bottom surface 14 and the skin 13 of the patient.

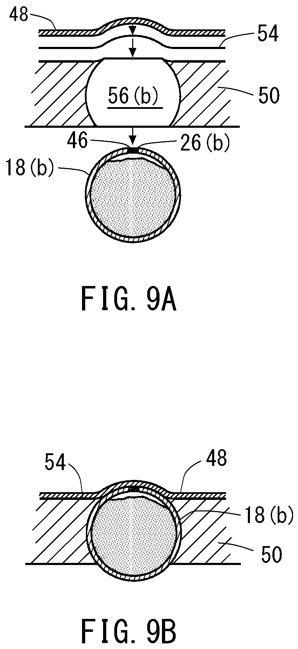

Fig. 9A is a cross-sectional view of the second subassembly from Fig. 8, as viewed along the cut-plane 9A-9A of Fig. 8, illustrating the receptacle 18(b) being assembled into one of the recesses 56(a)-(i) in the clamp sheet 50. The release liner 48 is illustrated above the clamp sheet 50.

Fig. 9B is a cross-sectional view similar to Fig. 9A except the receptacle 18(b) has been inserted into the recess 56(a)-(i) in the clamp sheet 50 and the release liner 48 has been attached to the clamp sheet 50. In certain embodiments, one or all of the components of the second subassembly are joined together by, for example, ultrasonic welding, bonding, clipping, or clamping.

In certain embodiments, the first subassembly of Fig. 7B and the second subassembly of Fig. 9B are joined together by, for example, ultrasonic welding, bonding, clipping, or clamping to form the final assembly of the patch 10 as illustrated in Fig. 4.

In certain embodiments, a method for manufacturing the patch 10 begins by providing the first subassembly as illustrated in Fig. 7B. The first subassembly has a bottom surface, a top surface, and the two or more receptacles 18(a)-(i) disposed between the bottom surface and the top surface. The bottom surface is configured for placement against the skin 13 of the patient. In certain embodiments, each receptacle 18 of the two or more receptacles 18(a)-(i) contains the solution or medication 44. The method continues by providing a second subassembly as illustrated in Fig. 9B. The second subassembly has the plurality of chambers 38(a)-(i). In certain embodiments, each chamber 38 of the plurality of chambers 38(a)-(i) includes one of the plurality of pyrotechnic charges 20(a)-(i). The method continues by joining the second subassembly to the top surface of the first subassembly so that the plurality of chambers 38(a)-(i) are on an opposite side of the two or more receptacles 18(a)-(i) from the bottom surface. In certain embodiments, the plurality of chambers 38(a)-(i) are aligned with the two or more receptacles 18(a)-(i) at least when the second subassembly is joined to the first subassembly. The method continues with the first subassembly and the second subassembly being joined together by, for example, ultrasonic welding, bonding, clipping, or clamping to form a final assembly.

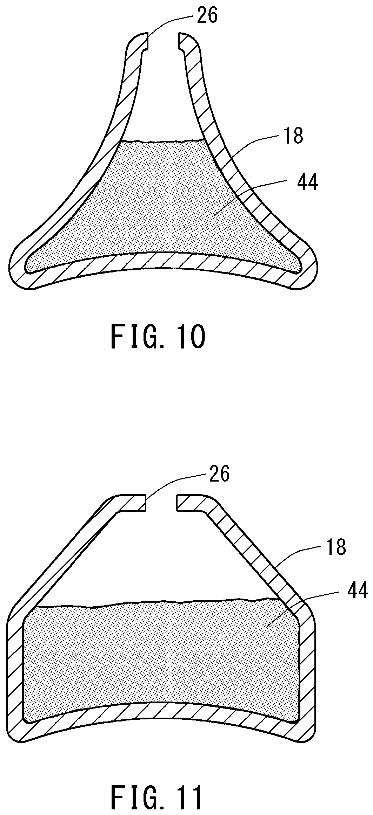

Fig. 10 illustrates another embodiment of the two or more receptacles 18(a)-(i) in Fig. 4 that has a truncated triangular shape. Fig. 11 illustrates another embodiment of the two or more receptacles 18(a)-(i) in Fig. 4 that has a truncated pentagon shape. Of course, the shape of the two or more receptacles 18(a)-(i) is not limited to the disclosed shapes and can instead have any shape.

The following supplementary note will be further provided for the embodiments described above.

### (Supplementary note 1)

A wearable multi-shot injection patch including:
a base having a bottom surface and a top surface, the bottom surface being configured for placement against skin of a patient;
a first receptacle supported by the base and containing a first medication;
a second receptacle supported by the base and containing a second medication;
a first pyrotechnic charge disposed in the base and between the top surface and the first receptacle;
a second pyrotechnic charge disposed in the base and between the top surface and the second receptacle; and
one or more electronic components configured to trigger the first pyrotechnic charge and the second pyrotechnic charge.

### (Supplementary note 2)

The wearable multi-shot injection patch according to supplementary note 1, further including:
one or more electronic components configured to trigger the first pyrotechnic charge and the second pyrotechnic charge;
a first nozzle supported by the base and disposed on an opposite side of the first receptacle from the first pyrotechnic charge; and
a second nozzle supported by the base and disposed on an opposite side of the second receptacle from the second pyrotechnic charge.

### (Supplementary note 3)

The wearable multi-shot injection patch according to supplementary note 2, in which
the first nozzle is positioned so that when the one or more electronic components trigger the first pyrotechnic charge, at least a portion of the first medication exits the first nozzle and penetrates the skin of the patient, and
the second nozzle is positioned so that when the one or more electronic components trigger the second pyrotechnic charge, at least a portion of the second medication exits the second nozzle and penetrates the skin of the patient.

### (Supplementary note 4)

The wearable multi-shot injection patch according to supplementary note 3, in which the first nozzle and the second nozzle are disposed relative to each so that the at least a portion of the first medication penetrates the skin of the patient at a location different from a location where the at least a portion of the second medication penetrates the skin of the patient.

### (Supplementary note 5)

The wearable multi-shot injection patch according to supplementary note 3, in which the first nozzle and the second nozzle are disposed relative to each so that the at least a portion of the first medication penetrates the skin of the patient at a same location as a location where the at least a portion of the second medication penetrates the skin of the patient.

### (Supplementary note 6)

The wearable multi-shot injection patch according to any one of supplementary note 1 to supplementary note 5, in which the base includes a circuit board, and is flexible so that a bottom surface of the circuit board can conform to a surface of a limb or torso of the patient.

### (Supplementary note 7)

The wearable multi-shot injection patch according to any one of supplementary note 1 to supplementary note 6, in which the base includes an adhesive layer configured to adhere the wearable multi-shot injection patch to the skin of the patient.

### (Supplementary note 8)

The wearable multi-shot injection patch according to any one of supplementary note 1 to supplementary note 7, further including a strap coupled to the base and configured to be secured around a limb or torso of the patient so as to maintain contact between the bottom surface and the skin of the patient.

### (Supplementary note 9)

The wearable multi-shot injection patch according to any one of supplementary note 1 to supplementary note 8, in which the one or more electronic components are configured to generate an electric current to trigger the first pyrotechnic charge and the second pyrotechnic charge.

### (Supplementary note 10)

The wearable multi-shot injection patch according to any one of supplementary note 1 to supplementary note 9, in which the one or more electronic components are configured to selectively trigger the first pyrotechnic charge and the second pyrotechnic charge.

### (Supplementary note 11)

The wearable multi-shot injection patch according to any one of supplementary note 1 to supplementary note 9, in which the one or more electronic components are configured to independently trigger each of the first pyrotechnic charge and the second pyrotechnic charge.

### (Supplementary note 12)

The wearable multi-shot injection patch according to any one of supplementary note 1 to supplementary note 9, in which the one or more electronic components are configured to simultaneously trigger both of the first pyrotechnic charge and the second pyrotechnic charge.

### (Supplementary note 13)

The wearable multi-shot injection patch according to any one of supplementary note 1 to supplementary note 12, in which the one or more electronic components are configured to be controlled remotely.

### (Supplementary note 14)

The wearable multi-shot injection patch according to any one of supplementary note 1 to supplementary note 12, in which the one or more electronic components are configured to be controlled in a predetermined timed sequence.

### (Supplementary note 15)

The wearable multi-shot injection patch according to any one of supplementary note 1 to supplementary note 14, in which the one or more electronic components include a microcontroller, the microcontroller being configured to store instructions to trigger at least one of the first pyrotechnic charge and the second pyrotechnic charge.

### (Supplementary note 16)

The wearable multi-shot injection patch according to supplementary note 15, in which the instructions specify an interval between triggering of the first pyrotechnic charge and the second pyrotechnic charge.

### (Supplementary note 17)

The wearable multi-shot injection patch according to supplementary note 15 or supplementary note 16, in which
the one or more electronic components include a timer, and
the microcontroller is configured to trigger at least one of the first pyrotechnic charge and the second pyrotechnic charge at least in part based on the timer.

### (Supplementary note 18)

The wearable multi-shot injection patch according to any one of supplementary note 1 to supplementary note 17, further including a battery configured to power the one or more electronic components.

### (Supplementary note 19)

The wearable multi-shot injection patch according to any one of supplementary note 1 to supplementary note 18, in which
the one or more electronic components include a receiver configured to receive a wireless signal, and
the one or more electronic components are configured to trigger at least one of the first pyrotechnic charge and the second pyrotechnic charge based at least in part on the wireless signal.

### (Supplementary note 20)

The wearable multi-shot injection patch according to supplementary note 19, in which the receiver is configured to receive the wireless signal from the patient.

### (Supplementary note 21)

The wearable multi-shot injection patch according to supplementary note 19, in which the receiver is configured to receive the wireless signal from a health professional.

### (Supplementary note 22)

The wearable multi-shot injection patch according to any one of supplementary note 1 to supplementary note 21, further including a user interface configured for the patient to initiate the one or more electronic components to trigger at least one of the first pyrotechnic charge and the second pyrotechnic charge.

### (Supplementary note 23)

The wearable multi-shot injection patch according to any one of supplementary note 1 to supplementary note 22, in which the base includes at least one first trace extending between the one or more electronic components and the first pyrotechnic charge.

### (Supplementary note 24)

The wearable multi-shot injection patch according to any one of supplementary note 1 to supplementary note 23, in which the base includes at least one first trace extending between the one or more electronic components and the second pyrotechnic charge.

### (Supplementary note 25)

The wearable multi-shot injection patch according to supplementary note 23, in which the base further includes a resistance wire in contact with the first pyrotechnic charge and electrically connected to the first trace.

### (Supplementary note 26)

The wearable multi-shot injection patch according to supplementary note 24, in which the base further includes a resistance wire in contact with the second pyrotechnic charge and electrically connected to the second trace.

### (Supplementary note 27)

The wearable multi-shot injection patch according to supplementary note 25 or supplementary note 26, in which the resistance wire has a diameter of 20 microns.

### (Supplementary note 28)

The wearable multi-shot injection patch according to any one of supplementary note 25 to supplementary note 27, in which the resistance wire has a length of 0.5 mm.

### (Supplementary note 29)

The wearable multi-shot injection patch according to any one of supplementary note 25 to supplementary note 28, in which the resistance wire has a resistance of 1 ohm.

### (Supplementary note 30)

The wearable multi-shot injection patch according to any one of supplementary note 1 to supplementary note 29, in which the first pyrotechnic charge and the second pyrotechnic charge include at least one of zirconium and potassium perchlorate.

### (Supplementary note 31)

The wearable multi-shot injection patch according to any one of supplementary note 1 to supplementary note 30, in which the first pyrotechnic charge and the second pyrotechnic charge include a dried slurry.

### (Supplementary note 32)

The wearable multi-shot injection patch according to any one of supplementary note 1 to supplementary note 31, in which
the base includes a mask layer forming a first chamber and a second chamber,
the first pyrotechnic charge is disposed in the first chamber, and the second pyrotechnic charge is disposed in the second chamber, and
at least a portion of the first chamber and at least a portion of the second chamber form a barrier between the first pyrotechnic charge and the second pyrotechnic charge.

### (Supplementary note 33)

The wearable multi-shot injection patch according to supplementary note 32, in which the mask layer includes a polymer material.

### (Supplementary note 34)

The wearable multi-shot injection patch according to supplementary note 32 or supplementary note 33, in which
the base further includes a sheet disposed between the mask layer and both the first receptacle and the second receptacle, and
the sheet has a first diaphragm positioned to cover the first chamber and a second diaphragm positioned to cover the second chamber.

### (Supplementary note 35)

The wearable multi-shot injection patch according to supplementary note 34, in which the sheet and the mask layer are joined together by one of ultrasonic welding, bonding, clipping and clamping.

### (Supplementary note 36)

The wearable multi-shot injection patch according to supplementary note 34 or supplementary note 35, in which each of the first diaphragm and the second diaphragm has a dome shape.

### (Supplementary note 37)

The wearable multi-shot injection patch according to supplementary note 36, in which each of the first receptacle and the second receptacle has a spherical shape.

### (Supplementary note 38)

The wearable multi-shot injection patch according to any one of supplementary note 34 to supplementary note 37, in which
the first diaphragm is flexible so that when the one or more electronic components trigger the first pyrotechnic charge, the first pyrotechnic charge generates a pressure spike to move the first diaphragm toward the first receptacle so that at least a portion of the first medication is ejected from the first receptacle, and
the second diaphragm is flexible so that when the one or more electronic components trigger the second pyrotechnic charge, the second pyrotechnic charge generates a pressure spike to move the second diaphragm toward the second receptacle so that at least a portion of the second medication is ejected from the second receptacle.

### (Supplementary note 39)

The wearable multi-shot injection patch according to any one of supplementary note 1 to supplementary note 38, in which the fist receptacle and the second receptacle are disposed at least partially within the base.

### (Supplementary note 40)

The wearable multi-shot injection patch according to supplementary note 2, further including:
an outer wall forming the first receptacle and having a first opening, the first nozzle being disposed in the first opening; and
an outer wall forming the second receptacle and having a second opening, the second nozzle being disposed in the second opening.

### (Supplementary note 41)

The wearable multi-shot injection patch according to supplementary note 2 or supplementary note 40, in which
the first nozzle is sized and shaped so that at least a portion of the first medication exiting the first nozzle forms a jet of liquid having sufficient velocity to penetrate the skin of the patient at a first location to a desired depth, and
the second nozzle is sized and shaped so that at least a portion of the second medication exiting the second nozzle forms a jet of liquid having sufficient velocity to penetrate the skin of the patient at a second location to a desired depth.

### (Supplementary note 42)

The wearable multi-shot injection patch according to any one of supplementary note 1 to supplementary note 41, in which
the base further includes a clamp sheet, and
the first receptacle and the second receptacle are disposed in the clamp sheet.

### (Supplementary note 43)

The wearable multi-shot injection patch according to any one of supplementary note 1 to supplementary note 42, in which the first medication is the same as the second medication.

### (Supplementary note 44)

The wearable multi-shot injection patch according to any one of supplementary note 1 to supplementary note 43, in which the first medication is different from the second medication.

### (Supplementary note 45)

The wearable multi-shot injection patch according to supplementary note 44, in which the first medication is complementary to the second medication.

### (Supplementary note 46)

The wearable multi-shot injection patch according to supplementary note 1 to supplementary note 45, in which the one or more electronic components simultaneously or sequentially trigger the first pyrotechnic charge and the second pyrotechnic charge.

### (Supplementary note 47)

A method for manufacturing a wearable multi-shot injection patch, including:
providing a first subassembly having a bottom surface and a top surface and including a plurality of receptacles disposed between the bottom surface and the top surface, the bottom surface being configured for placement against skin of a patient, each receptacle of the plurality of receptacles containing a medication;
providing a second subassembly including a plurality of chambers, each chamber of the plurality of chambers including a pyrotechnic charge; and
joining the second subassembly to the top surface of the first subassembly so that the plurality of chambers are on an opposite side of the plurality of receptacles from the bottom surface.

### (Supplementary note 48)

The method for manufacturing the multi-shot injection patch according to supplementary note 47, in which the plurality of chambers are aligned with the plurality of receptacles at least when the second subassembly is joined to the first subassembly.

### (Supplementary note 49)

The method for manufacturing the multi-shot injection patch according to supplementary note 47 or supplementary note 48, in which the second subassembly is joined to the first subassembly by one of ultrasonic welding, bonding, clipping and clamping.

### (Supplementary note 50)

A method for injecting a medication through skin of a patient using a wearable patch having a bottom surface and a top surface, including:
adhering the bottom surface of the wearable patch to the skin of the patient, the patch including a plurality of receptacles and a plurality of chambers aligned with the plurality of receptacles, the plurality of receptacles being disposed between the bottom surface and the top surface with each receptacle of the plurality of the receptacles containing the medication, each chamber of the plurality of chambers being disposed between the plurality of receptacles and the top surface and including a pyrotechnic charge; and
triggering the pyrotechnic charge in at least one chamber of the plurality of chambers to compress at least one receptacle of the plurality of receptacles so as to eject the medication from the at least receptacle and through the skin of the patient.

### <Terminology>

Although certain embodiments and examples are disclosed herein, inventive subject matter extends beyond the examples in the specifically disclosed embodiments to other alternative embodiments and/or uses, and to modifications and equivalents thereof. Thus, the scope of the claims appended hereto is not limited by any of the particular embodiments described above. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Thus, for example, various embodiments may be carried out in a manner that achieves or optimizes one aspect, advantage, or group of advantages as taught herein without necessarily achieving other aspects or advantages as may also be taught or suggested herein.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. Not necessarily all such aspects or advantages are achieved by any particular embodiment. For example, one or more of the features including 1) a receptacle for the medication; 2) a nozzle in the receptacle; 3) an outer wall forming the receptacle; 4) an opening in the receptacle; 5) an exit that is smaller than the receptacle to accelerate the medication; 6) structure compressing the receptacle to eject the medication; 7) a diaphragm sheet; 8) structure that presses the diagram sheet to eject the medication; 9) a specific chamber geometry; 10) structure preventing the pyrotechnic charge from contacting the skin; and/or 11) structure preventing the pyrotechnic charge from mixing with the medication. Therefore, the protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order illustrated or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those illustrated in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

For expository purposes, the term "horizontal" as used herein is defined as a plane parallel to the plane or surface of the floor or ground of the area in which the device being described is used or the method being described is performed, regardless of its orientation. The term "floor" can be interchanged with the term "ground." The term "vertical" refers to a direction perpendicular to the horizontal as just defined. Terms such as "above," "below," "bottom," "top," "side," "higher," "lower," "upper," "over," and "under," are defined with respect to the horizontal plane.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without other input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

Conjunctive language such as the phrase "at least one of X, Y, and Z, "unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

Although the multi-shot patch has been disclosed in the context of certain embodiments and examples, it will be understood by those skilled in the art that the multi-shot patch and subassemblies extends beyond the specifically disclosed embodiments to other alternative embodiments.

Accordingly, it is intended that the scope of the multi-shot patch herein disclosed should not be limited by the particular disclosed embodiments described above, but should be determined only by a fair reading of the claims that follow.

### Reference Signs List

- 10: Patch
- 11: Injection device
- 12: Base
- 18: Receptacle
- 20: Pyrotechnic charge
- 22: Electronic component
- 26: Nozzle
- 28: Circuit board
- 32: Trace
- 36: Mask layer
- 38: Chamber
- 40: Sheet
- 42: Diaphragm
- 50: Clamp sheet

## Claims

1. A wearable multi-shot injection patch (10), comprising:
a base (12) having a bottom surface (14) and a top surface (16), the bottom surface (14) being configured for placement against skin (13) of a patient;
a first subassembly comprising a first chamber (38) and a second chamber (38);
a second subassembly comprising a first receptacle (18(a)-(i)) supported by the base (12) and containing a first medication (44); and
a second receptacle (18(a)-(i)) supported by the base (12) and containing a second medication (44);
the first chamber (38) comprising a first pyrotechnic charge (20) disposed in the base (12) and between the top surface (16) and the first receptacle (18(a)-(i)) and the first chamber (38) being aligned with the first receptacle (18(a)-(i);
the second chamber (38) comprising a second pyrotechnic charge (20) disposed in the base (12) and between the top surface (16) and the second receptacle (18(a)-(i)) and the second chamber (38) being aligned with the second receptacle (18(a)-(i));
wherein the second subassembly is joined to a bottom surface of the first subassembly so that the first chamber (38) and the second chamber (38) are on an opposite side of the first receptacle (18(a)-(i)) and the second receptacle (18(a)-(i)) from the bottom surface; and
the wearable multi-shot injection patch (10) further comprises one or more electronic components (22) configured to trigger the first pyrotechnic charge (20) and the second pyrotechnic charge (20).

2. The wearable multi-shot injection patch (10) according to claim 1, further comprising:
a first nozzle (26) supported by the base (12) and disposed on an opposite side of the first receptacle (18(a)-(i)) from the first pyrotechnic charge (20); and
a second nozzle (26) supported by the base (12) and disposed on an opposite side of the second receptacle (18(a)-(i)) from the second pyrotechnic charge (20).

3. The wearable multi-shot injection patch (10) according to claim 1 or 2, wherein the base (12) comprises a circuit board (28) and is flexible so that a bottom surface of the circuit board (28) can conform to a surface of a limb or torso of the patient.

4. The wearable multi-shot injection patch (10) according to any one of claims 1 to 3, wherein the base (12) comprises an adhesive layer (54) configured to adhere the wearable multishot injection patch (10) to the skin (13) of the patient.

5. The wearable multi-shot injection patch (10) according to any one of claims 1 to 4, further comprising a strap coupled to the base (12) and configured to be secured around a limb or torso of the patient so as to maintain contact between the bottom surface (14) and the skin (13) of the patient.

6. The wearable multi-shot injection patch (10) according to any one of claims 1 to 5, wherein the one or more electronic components (22) are configured to selectively trigger the first pyrotechnic charge (20) and the second pyrotechnic charge (20) or simultaneously trigger both of the first pyrotechnic charge (20) and the second pyrotechnic charge (20).

7. The wearable multi-shot injection patch (10) according to any one of claims 1 to 6, wherein the one or more electronic components (22) are configured to be controlled remotely or controlled in a predetermined timed sequence.

8. The wearable multi-shot injection patch (10) according to any one of claims 1 to 7, wherein the one or more electronic components (22) comprise a microcontroller, the microcontroller being configured to store instructions to trigger at least one of the first pyrotechnic charge (20) and the second pyrotechnic charge (20).

9. The wearable multi-shot injection patch (10) according to any one of claims 1 to 8, wherein the one or more electronic components (22) comprise a receiver configured to receive a wireless signal, and
the one or more electronic components (22) are configured to trigger at least one of the first pyrotechnic charge (20) and the second pyrotechnic charge (20) based at least in part on the wireless signal.

10. The wearable multi-shot injection patch (10) according to any one of claims 1 to 9, wherein the base (12) comprises a mask layer (36) forming a first chamber (38) and a second chamber (38),
and
at least a portion of the first chamber (38) and at least a portion of the second chamber (38) form a barrier between the first pyrotechnic charge (20) and the second pyrotechnic charge (20).

11. The wearable multi-shot injection patch (10) according to claim 10, wherein
the base (12) further comprises a sheet (40) disposed between the mask layer (36) and both the first receptacle (18(a)-(i)) and the second receptacle (18(a)-(i)), and
the sheet (40) has a first diaphragm (42) positioned to cover the first chamber (38) and a second diaphragm (42) positioned to cover the second chamber (38).

12. The wearable multi-shot injection patch (10) according to claim 11, wherein each of the first diaphragm (42) and the second diaphragm (42) has a dome shape.

13. The wearable multi-shot injection patch (10) according to claim 12, wherein each of the first receptacle (18(a)-(i)) and the second receptacle (18(a)-(i)) has a spherical shape.

14. The wearable multi-shot injection patch (10) according to any one of claims 11 to 13, wherein
the first diaphragm (42) is flexible so that when the one or more electronic components (22) trigger the first pyrotechnic charge (20), the first pyrotechnic charge (20) generates a pressure spike to move the first diaphragm (42) toward the first receptacle (18(a)-(i)) so that at least a portion of the first medication (44) is ejected from the first receptacle (18(a)-(i)), and
the second diaphragm (42) is flexible so that when the one or more electronic components (22) trigger the second pyrotechnic charge (20), the second pyrotechnic charge (20) generates a pressure spike to move the second diaphragm (42) toward the second receptacle (18(a)-(i)) so that at least a portion of the second medication (44) is ejected from the second receptacle (18(a)-(i)).

15. The wearable multi-shot injection patch (10) according to any one of claims 1 to 14, wherein the base (12) further comprises a clamp sheet (50), and
the first receptacle (18(a)-(i)) and the second receptacle (18(a)-(i)) are disposed in the clamp sheet (50).

16. A method for manufacturing a wearable multi-shot injection patch (10), comprising:
providing a first subassembly comprising a plurality of chambers (38), each chamber (38) of the plurality of chambers (38) comprising a pyrotechnic charge (20);
providing a second subassembly having a bottom surface (14) and a top surface and comprising a plurality of receptacles (18(a)-(i)) disposed between the bottom surface (14) and the top surface,
the bottom surface (14) being configured for placement against skin (13) of a patient, each receptacle (18(a)-(i)) of the plurality of receptacles (18(a)-(i)) containing a medication (44);
and
joining the second subassembly to a bottom surface of the first subassembly so that the plurality of chambers (38) are on an opposite side of the plurality of receptacles (18(a)-(i)) from the bottom surface;
wherein the plurality of chambers (38) are aligned with the plurality of receptacles (18(a)-(i)) at least when the second subassembly is joined to the first subassembly.

## Patentansprüche

1. Tragbares Mikronadel-Pflaster (10), das umfasst:
einen Träger (12) mit einer unteren Fläche (14) und einer oberen Fläche (16), obei die untere Fläche (14) zur Aufbringung auf die Haut (13) eines Patienten ausgeführt ist;
eine erste Teilbaugruppe, die eine erste Kammer (38) sowie eine zweite Kammer (38) umfasst;
eine zweite Teilbaugruppe, die eine erste Aufnahme (18(a)-(i)) umfasst, die von dem Träger (12) getragen wird und ein erstes Medikament (44) enthält; sowie
eine zweite Aufnahme (18(a)-(i)), die von dem Träger (12) getragen wird und ein zweites Medikament (44) enthält;
wobei die erste Kammer (38) eine erste pyrotechnische Ladung (20) umfasst, die in dem Träger zwischen der oberen Fläche (16) und der ersten Aufnahme (18(a)-(i)) angeordnet ist, und die erste Kammer (38) mit der ersten Aufnahme (18(a)-(i)) fluchtend ist;
wobei die zweite Kammer (38) eine zweite pyrotechnische Ladung (20) umfasst, die in dem Träger zwischen der oberen Fläche (16) und der zweiten Aufnahme (18(a)-(i)) angeordnet ist, und die zweite Kammer (38) mit der zweiten Aufnahme (18(a)-(i)) fluchtend ist;
wobei die zweite Teilbaugruppe mit einer unteren Fläche der ersten Teilbaugruppe so verbunden ist, dass die erste Kammer (38) und die zweite Kammer (38) an einer der unteren Fläche gegenüberliegenden Seite der ersten Aufnahme (18(a)-(i)) und der zweiten Aufnahme (18(a)-(i)) liegen; und
das tragbare Mikronadel-Pflaster (10) des Weiteren eine oder mehrere elektronische Komponente/n (22) umfasst, die so ausgeführt ist/sind, dass sie die erste pyrotechnische Ladung (20) und die zweite pyrotechnische Ladung (20) zündet/zünden.

2. Tragbares Mikronadel-Pflaster (10) nach Anspruch 1, das des Weiteren umfasst:
eine erste Düse (26), die von dem Träger (12) getragen wird und an einer der ersten pyrotechnischen Ladung (20) gegenüberliegenden Seite der ersten Aufnahme (18(a)-(i)) angeordnet ist; sowie
eine zweite Düse (26), die von dem Träger (12) getragen wird und an einer der zweiten pyrotechnischen Ladung (20) gegenüberliegenden Seite der zweiten Aufnahme (18(a)-(i)) angeordnet ist.

3. Tragbares Mikronadel-Pflaster (10) nach Anspruch 1 oder 2, wobei der Träger (12) eine Leiterplatte (28) umfasst und flexibel ist, so dass sich eine untere Fläche der Leiterplatte (28) an eine Oberfläche eines Körpergliedes oder Rumpfes des Patienten anpassen kann.

4. Tragbares Mikronadel-Pflaster (10) nach einem der Ansprüche 1 bis 3, wobei der Träger (12) eine Klebeschicht (54) umfasst, die zum Ankleben des tragbaren Mikronadel-Pflasters (10) an der Haut (13) des Patienten ausgeführt ist.

5. Tragbares Mikronadel-Pflaster (10) nach einem der Ansprüche 1 bis 4, das des Weiteren ein Band umfasst, das mit dem Träger (12) verbunden und so ausgeführt ist, dass es um ein Körperglied oder einen Rumpf des Patienten herum befestigt wird, um Kontakt zwischen der unteren Fläche (14) und der Haut (13) des Patienten aufrechtzuerhalten.

6. Tragbares Mikronadel-Pflaster (10) nach einem der Ansprüche 1 bis 5, wobei die eine oder die mehreren elektronische/n Komponente/n (22) so ausgeführt ist/sind, dass sie selektiv die erste pyrotechnische Ladung (20) und die zweite pyrotechnische Ladung (20) zündet/zünden oder gleichzeitig sowohl die erste pyrotechnische Ladung (20) als auch die zweite pyrotechnische Ladung (20) zündet/zünden.

7. Tragbares Mikronadel-Pflaster (10) nach einem der Ansprüche 1 bis 6, wobei die eine oder die mehreren elektronische/n Komponente/n (22) so ausgeführt ist/sind, dass sie ferngesteuert wird/werden oder in einer vorgegebenen zeitlichen Sequenz gesteuert wird/werden.

8. Tragbares Mikronadel-Pflaster (10) nach einem der Ansprüche 1 bis 7, wobei die eine oder die mehreren elektronische/n Komponente/n (22) einen Mikrocontroller umfasst/umfassen, und der Mikrocontroller so ausgeführt ist, dass er Befehle zum Zünden der ersten pyrotechnischen Ladung (20) oder/und der zweiten pyrotechnischen Ladung (20) speichert.

9. Tragbares Mikronadel-Pflaster (10) nach einem der Ansprüche 1 bis 8, wobei die eine oder die mehreren elektronische/n Komponente/n (22) einen Empfänger umfasst/umfassen, der zum Empfangen eines Drahtlossignals ausgeführt ist, und
die eine oder die mehreren elektronische/n Komponente/n (22) so konfiguriert ist/sind, dass sie die erste pyrotechnische Ladung (20) oder/und die zweite pyrotechnische Ladung (20) wenigstens teilweise auf Basis des Drahtlossignals zündet/zünden.

10. Tragbares Mikronadel-Pflaster (10) nach einem der Ansprüche 1 bis 9, wobei der Träger (12) eine Maskenschicht (36) umfasst, die eine erste Kammer (38) sowie eine zweite Kammer (38) bildet,
und
wenigstens ein Abschnitt der ersten Kammer (38) sowie wenigstens ein Abschnitt der zweiten Kammer (38) eine Barriere zwischen der ersten pyrotechnischen Ladung (20) und der zweiten pyrotechnischen Ladung(20) bilden.

11. Tragbares Mikronadel-Pflaster (10) nach Anspruch 10, wobei
der Träger (12) des Weiteren eine Platte (40) umfasst, die zwischen der Maskenschicht (36) und sowohl der ersten Aufnahme (18(a)-(i)) als auch der zweiten Aufnahme (18(a)-(i)) angeordnet ist, und
die Platte (40) eine erste Membran (42), die so positioniert ist, dass sie die erste Kammer (38) abdeckt, und eine zweite Membran (42) aufweist, die so positioniert ist, dass sie die zweite Kammer (38) abdeckt.

12. Tragbares Mikronadel-Pflaster (10) nach Anspruch 11, wobei die erste Membran (42) und die zweite Membran (42) jeweils eine Kuppelform haben.

13. Tragbares Mikronadel-Pflaster (10) nach Anspruch 12, wobei die erste Aufnahme (18(a)-(i)) und die zweite Aufnahme (18(a)-(i)) jeweils eine Kugelform haben.

14. Tragbares Mikronadel-Pflaster (10) nach einem der Ansprüche 11 bis 13, wobei
die erste Membran (42) flexibel ist, so dass, wenn die eine oder die mehreren elektronische/n Komponente/n (22) die erste pyrotechnische Ladung (20) zündet/zünden, die erste pyrotechnische Ladung (20) eine Druckspitze erzeugt, durch die die erste Membran (42) in Richtung der ersten Aufnahme (18(a)-(i)) bewegt wird, so dass wenigstens ein Teil des ersten Medikaments (44) über die erste Aufnahme (18(a)-(i)) ausgestoßen wird, und
die zweite Membran (42) flexibel ist, so dass, wenn die eine oder die mehreren elektronische/n Komponente/n (22) die zweite pyrotechnische Ladung (20) zündet/zünden, die zweite pyrotechnische Ladung (20) eine Druckspitze erzeugt, durch die die zweite Membran (42) in Richtung der zweiten Aufnahme (18(a)-(i)) bewegt wird, so dass wenigstens ein Teil des zweiten Medikaments (44) über die zweite Aufnahme (18(a)-(i)) ausgestoßen wird.

15. Tragbares Mikronadel-Pflaster (10) nach einem der Ansprüche 1 bis 14, wobei der Träger (12) des Weiteren eine Klemmscheibe (50) umfasst, und
die erste Aufnahme (18(a)-(i)) und die zweite Aufnahme (18(a)-(i)) in der Klemmscheibe (50) angeordnet sind.

16. Verfahren zum Herstellen eines tragbaren Mikronadel-Pflasters (10), das umfasst:
Bereitstellen einer ersten Teilbaugruppe, die eine Vielzahl von Kammern (38) umfasst, wobei jede Kammer (38) der Vielzahl von Kammern (38) eine pyrotechnische Ladung (20) umfasst;
Bereitstellen einer zweiten Teilbaugruppe, die eine untere Fläche (14) sowie eine obere Fläche aufweist und eine Vielzahl von Aufnahmen (18(a)-(i)) umfasst, die zwischen der unteren Fläche (14) und der oberen Fläche angeordnet sind, die untere Fläche (14) zum Auflegen auf Haut (13) eines Patienten ausgeführt ist und jede Aufnahme (18(a)-(i)) der Vielzahl von Aufnahmen (18(a)-(i)) ein Medikament (44) enthält;
sowie
Verbinden der zweiten Teilbaugruppe mit einer unteren Fläche der ersten Teilbaugruppe so, dass sich die Vielzahl von Kammern (38) an einer der unteren Fläche gegenüberliegenden Seite der Vielzahl von Aufnahmen (18(a)-(i)) befinden;
wobei die Vielzahl von Kammern (38) mit der Vielzahl von Aufnahmen (18(a)-(i)) wenigstens dann fluchtend sind, wenn die zweite Teilbaugruppe mit der ersten Teilbaugruppe verbunden ist.

## Revendications

1. Timbre d'injection multi-shot portable (10), comprenant :
une base (12) ayant une surface inférieure (14) et une surface supérieure (16), la surface inférieure (14) étant configurée pour la mise en place contre la peau (13) d'un patient ;
un premier sous-ensemble comprenant une première chambre (38) et une seconde chambre (38) ;
un second sous-ensemble comprenant un premier réceptacle (18(a)-(i)) supporté par la base (12) et contenant un premier médicament (44) ; et
un second réceptacle (18(a)-(i)) supporté par la base (12) et contenant un second médicament (44) ;
la première chambre (38) comprenant une première charge pyrotechnique (20) disposée dans la base (12) et entre la surface supérieure (16) et le premier réceptacle (18(a)-(i)) et la première chambre (38) étant alignée sur le premier réceptacle (18(a)-(i)) ;
la seconde chambre (38) comprenant une seconde charge pyrotechnique (20) disposée dans la base (12) et entre la surface supérieure (16) et le second réceptacle (18(a)-(i)) et la seconde chambre (38) étant alignée sur le second réceptacle (18(a)-(i)) ;
dans lequel le second sous-ensemble est joint à une surface inférieure du premier sous-ensemble de sorte que la première chambre (38) et la seconde chambre (38) se trouvent sur un côté opposé du premier réceptacle (18(a)-(i)) et du second réceptacle (18(a)-(i)) depuis la surface inférieure ; et
le timbre d'injection multi-shot portable (10) comprend en outre un ou plusieurs composants électroniques (22) configurés pour déclencher la première charge pyrotechnique (20) et la seconde charge pyrotechnique (20).

2. Timbre d'injection multi-shot portable (10) selon la revendication 1, comprenant en outre :
une première buse (26) supportée par la base (12) et disposée sur un côté opposé du premier réceptacle (18(a)-(i)) depuis la première charge pyrotechnique (20) ; et
une seconde buse (26) supportée par la base (12) et disposée sur un côté opposé du second réceptacle (18(a)-(i)) depuis la seconde charge pyrotechnique (20).

3. Timbre d'injection multi-shot portable (10) selon la revendication 1 ou 2, dans lequel la base (12) comprend une carte de circuit (28) et est souple de sorte qu'une surface inférieure de la carte de circuit (28) peut se conformer à une surface d'un membre ou d'un torse du patient.

4. Timbre d'injection multi-shot portable (10) selon l'une quelconque des revendications 1 à 3, dans lequel la base (12) comprend une couche adhésive (54) configurée pour faire adhérer le timbre d'injection multi-shot portable (10) à la peau (13) du patient.

5. Timbre d'injection multi-shot portable (10) selon l'une quelconque des revendications 1 à 4, comprenant en outre une sangle accouplée à la base (12) et configurée pour être solidement fixée autour d'un membre ou d'un torse du patient afin de maintenir un contact entre la surface inférieure (14) et la peau (13) du patient.

6. Timbre d'injection multi-shot portable (10) selon l'une quelconque des revendications 1 à 5, dans lequel les un ou plusieurs composants électroniques (22) sont configurés pour déclencher sélectivement la première charge pyrotechnique (20) et la seconde charge pyrotechnique (20) ou déclencher simultanément à la fois la première charge pyrotechnique (20) et la seconde charge pyrotechnique (20).

7. Timbre d'injection multi-shot portable (10) selon l'une quelconque des revendications 1 à 6, dans lequel les un ou plusieurs composants électroniques (22) sont configurés pour être contrôlés à distance ou contrôlés sur une séquence de temps prédéterminée.

8. Timbre d'injection multi-shot portable (10) selon l'une quelconque des revendications 1 à 7, dans lequel les un ou plusieurs composants électroniques (22) comprennent un microcontrôleur, le microcontrôleur étant configuré pour stocker des instructions pour déclencher au moins l'une de la première charge pyrotechnique (20) et de la seconde charge pyrotechnique (20).

9. Timbre d'injection multi-shot portable (10) selon l'une quelconque des revendications 1 à 8, dans lequel les un ou plusieurs composants électroniques (22) comprennent un récepteur configuré pour recevoir un signal sans fil, et
les un ou plusieurs composants électroniques (22) sont configurés pour déclencher au moins l'une de la première charge pyrotechnique (20) et de la seconde charge pyrotechnique (20) sur la base au moins en partie du signal sans fil.

10. Timbre d'injection multi-shot portable (10) selon l'une quelconque des revendications 1 à 9, dans lequel la base (12) comprend une couche de masquage (36) formant une première chambre (38) et une seconde chambre (38), et
au moins une portion de la première chambre (38) et au moins une portion de la seconde chambre (38) forment une barrière entre la première charge pyrotechnique (20) et la seconde charge pyrotechnique (20).

11. Timbre d'injection multi-shot portable (10) selon la revendication 10, dans lequel
la base (12) comprend en outre une feuille (40) disposée entre la couche de masquage (36) et à la fois le premier réceptacle (18(a)-(i)) et le second réceptacle (18(a)-(i)), et
la feuille (40) présente un premier diaphragme (42) positionné pour recouvrir la première chambre (38) et un second diaphragme (42) positionné pour recouvrir la seconde chambre (38).

12. Timbre d'injection multi-shot portable (10) selon la revendication 11, dans lequel chacun du premier diaphragme (42) et du second diaphragme (42) présente une forme de dôme.

13. Timbre d'injection multi-shot portable (10) selon la revendication 12, dans lequel chacun du premier réceptacle (18(a)-(i)) et du second réceptacle (18(a)-(i)) présente une forme sphérique.

14. Timbre d'injection multi-shot portable (10) selon l'une quelconque des revendications 11 à 13, dans lequel le premier diaphragme (42) est souple de sorte que lorsque les un ou plusieurs composants électroniques (22) déclenchent la première charge pyrotechnique (20), la première charge pyrotechnique (20) génère un pic de pression pour déplacer le premier diaphragme (42) vers le premier réceptacle (18(a)-(i)) de sorte qu'au moins une portion du premier médicament (44) est éjectée du premier réceptacle (18(a)-(i)), et
le second diaphragme (42) est souple de sorte que lorsque les un ou plusieurs composants électroniques (22) déclenchent la seconde charge pyrotechnique (20), la seconde charge pyrotechnique (20) génère un pic de pression pour déplacer le second diaphragme (42) vers le second réceptacle (18(a)-(i)) de sorte qu'au moins une portion du second médicament (44) est éjectée du second réceptacle (18(a)-(i)).

15. Timbre d'injection multi-shot portable (10) selon l'une quelconque des revendications 1 à 14, dans lequel la base (12) comprend en outre une feuille de serrage (50), et
le premier réceptacle (18(a)-(i)) et le second réceptacle (18(a)-(i)) sont disposés dans la feuille de serrage (50).

16. Procédé de fabrication d'un timbre d'injection multi-shot portable (10), comprenant les étapes consistant à :
fournir un premier sous-ensemble comprenant une pluralité de chambres (38), chaque chambre (38) de la pluralité de chambres (38) comprenant une charge pyrotechnique (20) ;
fournir un second sous-ensemble ayant une surface inférieure (14) et une surface supérieure et comprenant une pluralité de réceptacles (18(a)-(i)) disposés entre la surface inférieure (14) et la surface supérieure, la surface inférieure (14) étant configurée pour la mise en place contre peau (13) d'un patient, chaque réceptacle (18(a)-(i)) de la pluralité de réceptacles (18(a)-(i)) contenant un médicament (44) ; et
à joindre le second sous-ensemble à une surface inférieure du premier sous-ensemble de sorte que la pluralité de chambres (38) se trouvent sur un côté opposé de la pluralité de réceptacles (18(a)-(i)) depuis la surface inférieure ;
dans lequel la pluralité de chambres (38) sont alignées sur la pluralité de réceptacles (18(a)-(i)) au moins lorsque le second sous-ensemble est joint au premier sous-ensemble.
